# FASCICULE DE BREVET EUROPEEN

(11) **EP 2 234 585 B1**
(45) Date de publication et mention de la délivrance du brevet: **14.11.2018**
(21) Numéro de dépôt: 08863702.0
(22) Date de dépôt: 02.12.2008
(51) Int. Cl.: A61K 8/25, A61Q 1/06, C09B 7/00, C09B 67/00, B82Y 30/00, C09C 1/42

(54) **COMPOSITION COSMETIQUE COMPRENANT UNE MATIERE COLORANTE ET PROCEDE DE TRAITEMENT COSMETIQUE**
KOSMETIKZUSAMMENSETZUNG MIT EINEM FARBSTOFF UND KOSMETISCHES BEHANDLUNGSVERFAHREN
COSMETIC COMPOSITION COMPRISING A DYESTUFF AND COSMETIC TREATMENT METHOD

(30) Priorité: 20.12.2007 FR 0760112; 22.01.2008 US 22524
(43) Date de publication de la demande: 06.10.2010
(73) Titulaire: L'Oréal, 75008 Paris (FR)
(72) Inventeur: RODRIGUEZ, Ivan, F-60290 Cauffry (FR); JEANNE-ROSE, Valérie, F-95100 Argenteuil (FR)
(74) Mandataire: Kromer, Christophe
(86) Numéro de dépôt international: PCT/FR2008/052173
(87) Numéro de publication internationale: WO 2009/080927

(56) Documents cités:
- WO-A1-2009/112646
- DE-C1- 19 833 545
- JP-A- 5 051 537
- US-A1- 2004 011 254
- US-A1- 2006 200 917
- US-B1- 6 333 026
- OLPHEN VAN H: "Maya blue: A clay-organic pigment?" SCIENCE, WASHINGTON, DC, vol. 154, 1 janvier 1966 (1966-01-01), pages 645-646, XP002440497 ISSN: 0036-8075

## Description

La présente invention concerne un procédé de traitement cosmétique employant des compositions cosmétiques comprenant de nouvelles matières colorantes.

Les compositions de maquillage contiennent généralement des matières colorantes, telles que pigments ou colorants, qui confèrent au maquillage déposé les couleurs recherchées. Le nombre de matières colorantes bleues utilisables en cosmétique est particulièrement limité. On connaît principalement des pigments ou colorants minéraux comme le bleu de Prusse ou l'ultramarine, et des pigments ou colorants organiques comme le bleu de phtalocyanine, le bleu de patenté (acid blue) et l'indigo. Les matières colorantes organiques présentent une force colonelle supérieure à celle des matières colorantes minérales, ce qui implique que l'on préfère leur utilisation à celle des matières colorantes minérales.
Cependant, on a constaté que lesdites matières colorantes organiques avaient tendance à se décolorer lorsqu'elles étaient soumises aux UV, ce qui n'est pas le cas des matières colorantes minérales, qui présentent une bonne stabilité aux UV.

Afin de remédier à ces problèmes de stabilité UV, il a été proposé de photostabiliser des colorants organiques, par l'ajout d'absorbants UV ou d'antioxydants, notamment par Daniela Cristea, Gerard Vilarem dans Dyes and Pigments 70 (2006) p238-245. Toutefois, ces additifs ne permettent pas de stabiliser la couleur de façon durable, c'est-à-dire pendant plusieurs mois; par ailleurs, la présence de certains additifs dans les compositions cosmétiques peut être indésirables, en fonction de la nature de ces additifs, ou peut générer des problèmes de formulation, tels que manque de stabilité, incompatibilité avec d'autres composants, etc.

La présente invention a pour but de proposer de nouvelles matières colorantes organiques, susceptibles d'être employées en cosmétique et présentant une bonne stabilité aux UV, durable pendant plusieurs mois.

Ainsi, la présente invention a pour objet un procédé cosmétique de maquillage des matières kératiniques, notamment de la peau du corps ou du visage, des lèvres, des ongles, des cheveux et/ou des cils, comprenant l'application sur lesdites matières d'une composition cosmétique comprenant, dans un milieu cosmétiquement acceptable, au moins une matière colorante comprenant une matrice inorganique fibreuse à tunnels et au moins un composé colorant organique incorporé au moins partiellement dans lesdits tunnels,
dans laquelle ledit composé colorant organique est choisi parmi les indigoïdes, et est présent dans la matière colorante en une quantité telle que le rapport pondéral composé colorant organique / matrice fibreuse inorganique est supérieur ou égal à 0,028 (soit 2,8%),
le composé colorant organique et la matrice fibreuse inorganique étant soumis à un traitement thermique effectué :
(i) soit en une seule étape, à une température supérieure ou égale à 320°C, pendant un temps supérieur ou égal à 15 minutes,
(ii) soit en une seule étape, à une température supérieure ou égale à 250°C, pendant un temps supérieur ou égal à 45 minutes ;
(iii) soit en deux étapes, la première étape étant effectuée à une température supérieure ou égale à 250°C, pendant un temps supérieur ou égal à 10 minutes, la seconde étape étant effectuée à une température supérieure ou égale à 250°C, pendant un temps supérieur ou égal à 5 minutes ;
(iv) soit en trois étapes, chaque étape étant effectuée à une température supérieure ou égale à 320°C, pendant un temps supérieur ou égal à 4 minutes.

Les matières colorantes selon l'invention peuvent présenter une gamme très variée de couleur, dans le domaine du bleu, allant du bleu au bleu-vert, en passant par le bleu foncé, le bleu turquoise ou le bleu indigo, ce qui permet de les utiliser de manière particulièrement avantageuse dans les compositions cosmétiques, notamment de maquillage.
Ceci est particulièrement remarquable et avantageux étant donné que ces matières colorantes présentent une composition de base quasi identique, ce qui permet de simplifier leur mise en oeuvre dans les compositions cosmétiques, lesdites matières colorantes étant interchangeables au sein d'une même composition de base, ceci permettant d'éviter les adaptations de chaque composition à la nature de chaque matière colorante.
Les matières colorantes selon l'invention présentent aussi comme avantage de ne pas dégorger dans les milieux cosmétiques usuels, qu'ils comprennent des huiles carbonées ou des huiles siliconées usuelles.

Les matières colorantes formées à partir d'indigo et d'argile sont connues dans l'art antérieur. On peut notamment citer les pigments connus dans la littérature sous la dénomination 'Bleus Maya' qui résultent de l'association d'indigo et d'une argile qui peut être du type palygorskyte ou sépiolite.
Le 'Bleu Maya' est un pigment bleu retrouvé dans plusieurs sites archéologiques, notamment au Mexique et au Guatemala. Il a notamment été retrouvé sur les peintures murales de Chichen Itza (1931, Merwin) et de Bonampak, au Mexique.
Il est remarquable par sa couleur bleue très particulière et également par son incroyable résistance, qui a permis de le retrouver sur des murs, des poteries, des objets de culte, datant du 16^{ème} siècle, en très bon état de conservation, et ce malgré des conditions de conservation, notamment climatiques, défavorables.

Concernant les pigments de type 'Bleu Maya' synthétiques, on peut notamment citer US7052541 qui décrit des matières colorantes susceptibles d'être utilisées dans le domaine des peintures, des plastiques ou des ciments, et qui sont obtenues à partir d'un dérivé d'indigo et d'une argile qui peut être fibreuse ou lamellaire.
On connaît également par US2006/0200917, un procédé particulier de préparation de matières colorantes destinées au domaine de la peinture ou du ciment, consistant à mélange un indigo ou un dérivé d'indigo, avec une argile fibreuse ou lamellaire, puis à soumettre le mélange à un rayonnement ultraviolet, notamment à une longueur d'ondes de 200-400 nm, pendant 1 minute à 48 heures.
Toutefois, aucun de ces documents ne décrit ni ne suggère l'utilisation de telles matières colorantes dans des compositions cosmétiques, notamment de maquillage, et encore moins l'utilisation en cosmétique des matières colorantes particulières telles que décrites dans la présente invention.
Par ailleurs, il n'est nullement mentionné dans ces documents que les matières colorantes présentent une bonne photostabilité.

Le document US6333026 B1 décrit un procédé cosmétique de maquillage des matières kératiniques comprenant l'application d'un composé indigoïde.
Les matières colorantes utilisées selon l'invention comprennent une matrice inorganique fibreuse à tunnels, et au moins un composé colorant organique incorporé au moins partiellement dans lesdits tunnels.
Par partiellement, on entend qu'au moins 30% en poids de la quantité totale de colorant organique est inclus dans les canaux de la matrice fibreuse.
L'incorporation du colorant organique dans ladite matrice organique peut notamment être illustrée par RMN, notamment par RMN 2D HECTOR à très haute vitesse de rotation MAS du 29Si, 13C et 1H du colorant organique; on peut ainsi déterminer la structure de la matrice fibreuse et la présence de colorant inclus (espèce 'in') et de colorant adsorbé en surface (espèce 'out'); en comparant les déplacement chimiques du spectre 13C du colorant organique tel quel, à celui du colorant organique dans la matière colorante finale, il est possible de caractériser l'incorporation dudit colorant dans ladite matrice. Enfin, la corrélation observée entre les protons du colorant organique et le site 1 de la matrice fibreuse dans l'expérience 2D HECTOR 1H-29Si offre une preuve tangible de l'inclusion dudit colorant dans ladite matrice.
On a en effet constaté qu'avec les procédés selon l'invention, il était possible d'obtenir des matières colorantes particulièrement photostables, ladite photostabilité étant notamment en partie due au fait que les composés colorants organiques sont au moins partiellement incorporés dans les tunnels de la matrice fibreuse.

Ladite matrice inorganique fibreuse à tunnels est de préférence une argile.
On sait que l'argile est une roche sédimentaire, composée pour une large part de minéraux spécifiques, silicates en général d'aluminium plus ou moins hydratés, qui présentent une structure feuilletée (ou lamellaire) ou bien une structure fibreuse. On les classe en trois grandes familles selon l'épaisseur des feuillets, qui correspondent à un nombre de couches d'oxydes tétraédriques et octaédriques.
L'interstice entre feuillets peut contenir de l'eau ainsi que des ions. Il en résulte des variations de la distance entre feuillets, et donc des variations dimensionnelles macroscopiques de l'argile quand elle s'hydrate ou s'assèche.
Les argiles utilisables dans le cadre de la présente invention sont des argiles fibreuses (ou à structure fibreuse), et notamment de type sépiolite ou palygorskite (aussi appelée attapulgite).
Les palygorskites et les sépiolites sont des argiles généralement constituées de fibres de l'ordre de 1 à 3 microns de long.
Dans la sépiolite, chaque fibre est formée d'une multitude de tunnels (ou canalicules) d'environ 1 nm² régulièrement espacés. Cette configuration particulière en briques creuses allongées, propre à son arrangement cristallin, lui confère une surface spécifique très importante. La formule générale retenue dans la littérature pour sa structure cristalline est Mg₄Si₆O₅(OH)₂.6(H₂O) ou encore plus préférentiellement (Si)₁₂(Mg)₈(O)₃₀(OH)₄(OH₂)₄.8H₂O
Dans le cadre de l'invention, on peut utiliser indifféremment la sépiolite ou la palygorskyte, ou encore un mélange de sépiolite et de palygorskyte, en toute proportion; et de préférence la sépiolite seule.

Les composés colorants organiques utilisés selon l'invention sont choisis parmi les indigoïdes, seuls ou en mélange entre eux.
De préférence, on utilise comme composé colorant organique l'indigo; c'est un colorant naturel, provenant notamment de l'indigotier et dont la formule brute est : C₁₆H₁₀N₂O₂; il a pour structure :

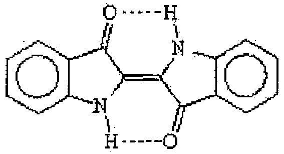

Il est possible d'utiliser, en mélange avec l'indigo, d'autres colorants organiques additionnels de la famille des indigoïdes, seuls ou en mélange entre eux, tels que l'indirubine, l'indigotine, les halogéno-indigo tels que le dichloroindigo, le dibromoindigo, les thioindigos, les acétates d'indigo.

De préférence, lorsqu'ils sont présents, ces colorants organiques additionnels représentent 0,01 à 25% en poids, notamment 0,1 à 20% en poids, voire 0,5 à 13% en poids, du mélange 'indigo + colorants additionnels indigoïdes'.

De préférence, les composés colorants organiques se présentent sous forme solide à 25°C; ils peuvent toutefois se présenter sous forme liquide à 25°C.

Avantageusement, les composés colorants organiques sont employés tels quels, c'est-à-dire en l'absence de solvant.

Pour pouvoir être incorporés dans les tunnels de la matrice fibreuse, les composés colorants organiques sont de préférence tels qu'au moins deux de leurs dimensions sont inférieures ou égales à, respectivement, 1,1 nm et 0,57 nm, la troisième dimension pouvant être quelconque. De préférence, l'une des dimensions est comprise entre 1,0 et 1,1 nm, notamment entre 1,04 et 1,08 nm; et/ou une autre dimension est comprise entre 0,25 et 0,45 nm, notamment entre 0,30 et 0,40 nm.

Les composés colorants organiques peuvent porter une ou plusieurs fonctions polaires, notamment thiol, qui peuvent faciliter leur incorporation dans les tunnels de la matrice fibreuse.

Afin de préparer les matières colorantes selon l'invention, on mélange la matrice inorganique fibreuse à tunnels et les composés colorants organiques, de manière à ce que lesdits composés colorants soient incorporés, au moins partiellement, dans les tunnels de la matrice fibreuse.

Le composé colorant organique est présent dans la matière colorante en une quantité telle que le rapport pondéral initial composé colorant organique (totaux, donc optionnels compris) / matrice fibreuse inorganique est supérieur ou égal à 0,028 (soit 2,8%), notamment compris entre 0,0285 (2,85%) et 0,20 (20%), encore mieux entre 0,029 (2,9%) et 0,15 (15%), voire entre 0,03 (3%) et 0,13 (13%), préférentiellement entre 0,032 (3,2%) et 0,08 (8%).

Afin d'obtenir un mélange particulièrement homogène, on peut effectuer une étape de mélange/malaxage, qui peut être effectuée dans un broyeur notamment de type mortier-pilon, ou encore dans une bétonnière.
L'étape optionnelle de mélange/malaxage n'a de préférence pas pour objet de diminuer la taille de la matrice fibreuse, la matrice fibreuse comprenant les composés colorants ayant de préférence une taille quasi-identique à celle de la matrice fibreuse initiale.
L'étape de mélange/malaxage peut être effectuée pendant 10 secondes à 72 heures, notamment 1 à 120 minutes, encore mieux 10 à 100 minutes. Elle est de préférence effectuée à température ambiante (20-30°C).

Le mélange comprenant la matrice inorganique fibreuse et les composés colorants organiques, éventuellement broyés/malaxés, est de préférence traité thermiquement à une température supérieure ou égale à 250°C, pendant un temps total supérieur ou égal à 12 minutes.
Le temps et la température de traitement thermique doivent être choisis de manière telle que les composés colorants organiques soient au final incorporés au moins partiellement dans la matrice inorganique fibreuse.
Ainsi, plus la température de traitement thermique sera élevée, plus le temps pourra est réduit. A l'inverse, plus la température sera faible, plus le temps de traitement thermique devra être élevé; ceci pour obtenir une incorporation adéquate des composés colorants dans la matrice, et donc une photostabilité telle que recherchée.
Le nombre d'étape de traitement thermique peut également être pris en compte; il est ainsi possible d'effectuer ledit traitement thermique en plusieurs étapes, notamment en deux, trois ou quatre étapes de chauffage. Il est possible de chauffer moins longtemps, et/ou à une température moins élevée, si l'on effectue le traitement thermique en plusieurs étapes.

Ainsi, le temps de traitement thermique est de préférence supérieur ou égal à 12 minutes, de préférence compris entre 12 et 120 minutes, notamment entre 15 et 90 minutes, voire entre 20 et 75 minutes; ce temps est le temps total de traitement thermique, c'est-à-dire la somme des temps de traitement thermique, lorsqu'il y a plusieurs étapes de chauffage.

La température de traitement thermique, quant à elle, est, pour chaque étape, supérieure ou égale à 250°C, de préférence comprise entre 250°C et 600°C, notamment entre 260°C et 575°C, voire entre 270°C et 550°C, préférentiellement entre 300°C et 500°C.

Dans un premier mode de réalisation préféré de l'invention, le traitement thermique peut être effectué en une seule étape, à une température supérieure ou égale à 320°C, notamment comprise entre 320°C et 600°C, de préférence comprise entre 340°C et 550°C, pendant un temps supérieur ou égal à 15 minutes, notamment compris entre 15 et 45 minutes, de préférence entre 18 et 40 minutes.

Dans un second mode de réalisation préféré de l'invention, le traitement thermique peut être effectué en une seule étape, à une température supérieure ou égale à 250°C, notamment comprise entre 250°C et 320°C, de préférence comprise entre 260°C et 300°C, pendant un temps supérieur ou égal à 45 minutes, notamment compris entre 45 et 120 minutes, de préférence entre 50 et 90 minutes.

Dans un troisième mode de réalisation préféré de l'invention, le traitement thermique peut être effectué en deux étapes,
la première étape étant effectuée à une température supérieure ou égale à 250°C, notamment comprise entre 250°C et 320°C, de préférence comprise entre 260°C et 300°C, pendant un temps supérieur ou égal à 10 minutes, notamment compris entre 10 et 120 minutes, de préférence entre 15 et 90 minutes;
la seconde étape étant effectuée à une température supérieure ou égale à 250°C, notamment comprise entre 250°C et 350°C, de préférence comprise entre 270°C et 320°C, pendant un temps supérieur ou égal à 5 minutes, notamment compris entre 5 et 90 minutes, de préférence entre 10 et 60 minutes.
De préférence, la température de la seconde étape est supérieure à celle de la première étape, notamment supérieure d'au moins 10°C.

Dans un quatrième mode de réalisation préféré de l'invention, le traitement thermique peut être effectué en trois étapes, chaque étape étant effectuée à une température supérieure ou égale à 320°C, notamment comprise entre 320°C et 600°C, de préférence comprise entre 340°C et 550°C, pendant un temps supérieur ou égal à 4 minutes, notamment compris entre 4 et 40 minutes, de préférence entre 5 et 30 minutes.

Le traitement thermique peut être effectué par l'homme du métier dans tout type de four; de préférence le mélange est introduit dans le four préalablement chauffé à la température de traitement souhaitée.
Lorsque le traitement thermique est effectué en plusieurs étapes, le mélange est de préférence laissé à refroidir jusqu'à température ambiante (25°C), à l'air libre, entre chaque étape.

On a constaté que le traitement thermique selon l'invention conduit à des matières colorantes très photostables, qui conservent donc leur couleur et leur force colorielle même après exposition pendant une longue période, à la lumière.

Par ailleurs, les matières colorantes utilisées selon l'invention présentent un très faible, voire inexistant, relarguage au chauffage, les composés colorants organiques étant stabilisés et immobilisés à long terme dans la matrice fibreuse inorganique.

Les matières colorantes selon l'invention peuvent être avantageusement utilisées dans le domaine cosmétique.

Elles peuvent être présentes dans les compositions cosmétiques à raison de 0,1 à 70% en poids, notamment 0,5 à 50% en poids, voire 1 à 40% en poids, préférentiellement 5 à 35% en poids, par rapport au poids total de la composition cosmétique.

Ladite composition cosmétique comprend par ailleurs un milieu cosmétiquement acceptable, c'est-à-dire un milieu compatible avec les tissus cutanés comme la peau du visage ou du corps, et les matières kératiniques telles que les cheveux, les cils, les sourcils et les ongles.

La composition peut alors comprendre, selon l'application envisagée, les constituants habituels à ce type de composition.
La composition selon l'invention peut avantageusement comprendre une phase grasse liquide, qui peut comprendre au moins un composé choisi parmi les huiles et/ou solvants d'origine minérale, animale, végétale ou synthétique, carbonés, hydrocarbonés, fluorés et/ou siliconés, volatils ou non volatils, seuls ou en mélange dans la mesure où ils forment un mélange homogène et stable et sont compatibles avec l'utilisation envisagée.
Par 'volatil', on entend au sens de l'invention, tout composé susceptible de s'évaporer au contact des matières kératiniques, ou des lèvres, en moins d'une heure, à température ambiante (25°C) et pression atmosphérique (1 atm). Notamment, ce composé volatil a une pression de vapeur non nulle, à température ambiante et pression atmosphérique, notamment allant de 0,13 Pa à 40 000 Pa (10⁻³ à 300 mm de Hg).
Par opposition, on entend par 'non volatil', un composé restant sur les matières kératiniques ou les lèvres à température ambiante et pression atmosphérique, au moins une heure et ayant notamment une pression de vapeur inférieure à 10⁻³ mm de Hg (0,13Pa).

De préférence, le milieu cosmétiquement acceptable de la composition selon l'invention peut comprendre, dans une phase grasse liquide, au moins une huile et/ou un solvant qui peut être choisi parmi, seul ou en mélange :
1/ les esters des acides monocarboxyliques avec les monoalcools et polyalcools; avantageusement, ledit ester est un benzoate d'alkyle en C12-C15 ou répond à la formule suivante : R'₁-COO-R'₂ où :
   R'₁ représente un radical alkyle linéaire ou ramifié de 1 à 40 atomes de carbone, de préférence de 7 à 19 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles, et
   R'₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles.
Par "éventuellement substitué", on entend que R'₁ et/ou R'₂ peuvent porter un ou plusieurs substituants choisis, par exemple, parmi les groupements comprenant un ou plusieurs hétéroatomes choisi parmi O et/ou N, tels que amino, amine, alcoxy, hydroxyle.
Des exemples des groupes R'₁ sont ceux dérivés des acides gras de préférence supérieur choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, oléostéarique, arachidonique, érucique, et de leurs mélanges.
De préférence, R'₁ est un groupe alkyle ramifié non substitué de 4 à 14 atomes de carbone, de préférence de 8 à 10 atomes de carbone et R₂ est un groupe alkyle ramifié non substitué de 5 à 15 atomes de carbone, de préférence de 9 à 11 atomes de carbone.

On peut en particulier citer, de préférence, les esters en C₈-C₄₈, éventuellement incorporant dans leur chaîne hydrocarbonée un ou plusieurs hétéroatomes parmi N et O et/ou une ou plusieurs fonctions carbonyle; et plus particulièrement l'huile de purcellin (octanoate de cétostéaryle), l'isononanoate d'isononyle, le myristate d'isopropyle, le palmitate d'isopropyle, le palmitate d'éthyl-2-hexyle, le stéarate d'octyl-2-dodécyle, l'érucate d'octyl-2-dodécyle, l'isostéarate d'isostéaryle, le benzoate d'alcool en C₁₂ à C₁₅, le laurate d'hexyle, l'adipate de diisopropyle; et les heptanoates, octanoates, décanoates ou ricinoléates d'alcools ou de polyalcools, par exemple d'alcools gras comme le dioctanoate de propylène glycol, ainsi que le N-lauroyl sarcosinate d'isopropyle (notamment Eldew-205SL d'Ajinomoto); les esters hydroxylés comme le lactate d'isostéaryle, le malate de di-isostéaryle; et les esters du pentaérythritol; les esters ramifiés en C8-C16, notamment le néopentanoate d'isohexyle.
2/ les huiles végétales hydrocarbonées à forte teneur en triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées; ces huiles sont notamment les huiles de germe de blé, de maïs, de tournesol, de karité, de ricin, d'amandes douces, de maca-damia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat, de jojoba, de palme, de calophyllum; ou encore les triglycérides des acides caprylique/caprique comme ceux vendus par la société Stearinerie Dubois ou ceux vendus sous les dénominations "Miglyol 810®", "812®" et "818®" par la société Dynamit Nobel.
3/ les alcools, et notamment les monoalcools, en C6-C32, notamment C12-C26, comme l'alcool oléïque, l'alcool linoléique, l'alcool linolénique, l'alcool isostéarylique, le 2-hexyldécanol, le 2-butyloctanol, le 2-undécylpentadécanol et l'octyldodécanol;
4/ les huiles hydrocarbonées, linéaires ou ramifiés, volatiles ou non, d'origine synthétique ou minérale, qui peuvent être choisies parmi les huiles hydrocarbonées ayant de 5 à 100 atomes de carbones, et notamment la vaseline, les polydécènes, les polyisobutènes hydrogénés tel que le Parléam, le squalane, le perhydrosqualène et leurs mélanges.
   On peut plus particulièrement citer les alcanes linéaires, ramifiés et/ou cycliques en C5-C48, et préférentiellement les alcanes ramifiés en C8-C16 comme les isoalcanes en C8-C16 d'origine pétrolière (appelées aussi isoparaffines); notamment le décane, l'heptane, le dodécane, le cyclohexane; ainsi que l'isododécane, l'isodécane, l'isohexadécane.
5/ les huiles de silicone, volatiles ou non volatiles;
   Comme huiles de silicone volatiles, on peut citer les huiles de silicones linéaires ou cycliques volatiles, notamment celles ayant une viscosité inférieure à 8 centistokes, et ayant notamment de 2 à 10 atomes de silicium, ces silicones comportant éventuellement des groupes alkyles ou alkoxy ayant de 1 à 22 atomes de carbone; et en particulier l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'hexaméthyldisiloxane, l'octaméthyltrisiloxane, le décaméthyltétrasiloxane, le dodécaméthylpentasiloxane, le méthylhexyldiméthylsiloxane et leurs mélanges.
   Les huiles de silicone non volatiles utilisables selon l'invention peuvent être les polydiméthylsiloxanes (PDMS), les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényltriméthicones, les phényldiméthicones, les phényltriméthylsiloxydiphénylsiloxanes, les diphényldiméthicones, les diphénylméthyldiphényltrisiloxanes, les 2-phényléthyl triméthylsiloxysilicates.

La phase grasse liquide peut en outre comprendre des huiles et/ou solvants additionnels, qui peuvent être choisis parmi, seul ou en mélange :
- les huiles fluorées telles que les perfluoropolyéthers, les perfluoroalcanes comme la perfluorodécaline, les perfluorodamantanes,les monoesters, diesters et triesters de perfluoroalkylphosphates et les huiles esters fluorés;
- les huiles d'origine animale;
- les éthers en C₆ à C₄₀, notamment en C10-C40; les éthers de propylène glycol liquides à température ambiante tels que le monométhyléther de propylène glycol, l'acétate de monométhyléther de propylène glycol, le mono n-butyl éther de dipropylène glycol ;
- les acides gras en C₈-C₃₂, comme l'acide oléique, l'acide linoléique, l'acide linolénique et leurs mélanges.
- les huiles bifonctionnelles, comprenant deux fonctions choisies parmi ester et/ou amide et comprenant de 6 à 30 atomes de carbone, notamment 8 à 28 atomes de carbone, mieux de 10 à 24 carbones, et 4 hétéroatomes choisis parmi O et N; de préférence les fonctions amide et ester étant dans la chaîne;
- les cétones liquides à température ambiante (25°C) tels que la méthyléthylcétone, la méthylisobutylcétone, la diisobutylcétone, l'isophorone, la cyclohexanone, l'acétone ;
- les aldéhydes liquides à température ambiante tels que le benzaldéhyde, l'acétaldéhyde;

La phase grasse liquide peut représenter 1 à 90% en poids de la composition, notamment de 5 à 75% en poids, en particulier de 10 à 60% en poids, voire de 25 à 55% en poids, du poids total de la composition.

La composition utilisée selon l'invention peut comprendre avantageusement un agent épaississant qui peut en particulier être choisi parmi :
- les silices notamment hydrophobes,
- les argiles telles que la montmorillonite, les argiles modifiées telles que les bentones par exemple, l'hectorite stéaralkonium, la bentonite stéaralkonium,
- les alkyléther de polysaccharides (notamment dont le groupe alkyle comporte de 1 à 24 atomes de carbones, de préférence de 1 à 10, mieux de 1 à 6, et plus spécialement de 1 à 3).

La quantité d'agent épaississant dans la composition selon l'invention peut aller de 0,05 à 40% en poids, par rapport au poids total de la composition, de préférence de 0,5 à 20% et mieux de 1 à 15% en poids.

La composition utilisée selon l'invention peut également comprendre au moins une cire d'origine végétale, animale, minérale ou de synthèse, voire siliconée.
On peut en particulier citer, seules ou en mélange, les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de Montan; les ozokérites; les cires de polyéthylène; les cires obtenues par synthèse de Fischer-Tropsch; les huiles hydrogénées, les esters gras et les glycérides concrets à 25°C. On peut également utiliser des cires de silicone, parmi lesquelles on peut citer les alkyls, alcoxys et/ou esters de polyméthylsiloxane.
La quantité de cire dans la composition selon l'invention peut aller de 0,1 à 70% en poids, par rapport au poids total de la composition, de préférence de 1 à 40% en poids, et mieux de 5 à 30% en poids.

La composition utilisée selon l'invention peut également comprendre une ou plusieurs matières colorantes additionnelles qui peuvent être choisies parmi les composés pulvérulents comme les pigments, les charges, les nacres et les paillettes, et/ou les colorants liposolubles ou hydrosolubles.
Les matières colorantes, notamment pulvérulentes, peuvent être présentes, dans la composition, en une teneur de 0,01 à 50% en poids, par rapport au poids de la composition, de préférence de 0,1 à 40% en poids, voire de 1 à 30% en poids.
Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.
Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.
Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
Les charges peuvent être minérales ou organiques, lamellaires ou sphériques. On peut citer le talc, le mica, la silice, le kaolin, les poudres de Nylon et de polyéthylène, de paly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydrocarbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium. Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter 0,01 à 20% du poids de la composition et mieux de 0,1 à 6 %.
Les colorants hydrosolubles sont par exemple le jus de betterave, le bleu de méthylène et peuvent représenter 0,01 à 6% du poids total de la composition.

La composition peut comprendre, en outre, d'autres ingrédients utilisés couramment dans les compositions cosmétiques. De tels ingrédients peuvent être choisis parmi les antioxydants, les parfums, les huiles essentielles, les conservateurs, les actifs cosmétiques, les hydratants, les vitamines, les céramides, les filtres solaires, les tensioactifs, les agents d'étalement, les agents mouillants, les agents dispersants, les antimousses, les neutralisants, les stabilisants, les polymères et notamment les polymères filmogènes liposolubles, et leurs mélanges.
Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Les compositions utilisées selon l'invention peuvent se présenter sous toute forme acceptable et usuelle pour une composition cosmétique ou pharmaceutique.
Elles peuvent donc se présenter sous la forme d'une suspension, une dispersion notamment d'huile dans de l'eau grâce à des vésicules; une solution organique ou huileuse éventuellement épaissie voire gélifiée; une émulsion huile-dans-eau, eau-dans-huile, ou multiple; un gel ou une mousse; un gel huileux ou émuisionné; une dispersion de vésicules notamment lipidiques; une lotion biphase ou multiphase; un spray; d'une lotion, d'une crème, d'une pommade, d'une pâte souple, d'un onguent, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Les compositions peuvent être utilisées pour le maquillage des matières kératiniques telles que les cheveux, la peau, les cils, les sourcils, les ongles, les lèvres, le cuir chevelu et plus particulièrement pour le maquillage des lèvres, des cils et/ou du visage.

Elles peuvent donc se présenter sous la forme d'un produit de maquillage de la peau du corps ou du visage, des lèvres, des cils, des sourcils, des cheveux, du cuir cheveu ou des ongles; elles se présentent avantageusement sous forme de composition de maquillage, notamment de mascara, de rouge à lèvres, de fard à joues, de fard à paupières, de fond de teint.

Les matières colorantes utilisées selon l'invention trouvent une application toute préférée dans des compositions de maquillage de type fard à paupières ou fard à joues.

Dans ce mode de réalisation, la composition utilisée selon l'invention comprend de préférence un milieu anhydre cosmétiquement acceptable, c'est-à-dire un milieu anhydre compatibles avec la peau des paupières. Ce milieu anhydre forme une phase continue. Par milieu "anhydre", on entend un milieu comprenant moins de 5% d'eau, et encore mieux moins de 1% d'eau.
Ce milieu anhydre peut comprendre en particulier au moins une huile qui est de préférence choisie parmi les huiles d'origine minérale, animale, végétale ou synthétique, hydrocarbonées et/ou siliconées, notamment tells que citées ci-dessus, et leurs mélanges. L'huile peut être présente dans la composition selon l'invention en une teneur allant de 0,1% à 60% en poids, par rapport au poids total de la composition, de préférence allant de 1% à 40% en poids, et préférentiellement allant de 5% à 25% en poids.
Ce milieu peut également comprendre des corps gras additionnels autres que les huiles, tels que les cires notamment citées ci-dessus, les pâteux ou les gommes. Comme corps gras pâteux, on peut citer des corps gras ayant un point de fusion allant de 25 à 45°C et/ou une viscosité à 40°C allant de 0,1 à 40 Pa.s mesurée au Contraves TV équipé d'un mobile MS-r3 ou Ms-r4 tournant à 60 Hz. A titre d'exemple de corps gras pâteux, on peut citer les lanolines et les dérivés de lanoline comme les lanolines acétylées ou les lanolines oxypropylènées, et leurs mélanges; des esters d'acides ou d'alcools gras, notamment ceux ayant 20 à 65 atomes de carbone comme le citrate de tri-isostéaryle ou de cétyle, le propionate d'arachidyle, le polylaurate de vinyle, les esters du cholestérol comme les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée, tels que le « THIXINR » de Rheox. On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stéaryl diméthicones; et leurs mélanges. Comme gommes, on peut utiliser les gommes de silicone (diméthiconols) comme par exemple le mélange dimethiconol/cyclopentasiloxane. Le corps gras additionnel peut être présent en une teneur de 0,1% à 30% en poids, par rapport au poids total de la composition, et de préférence de 1% à 15% en poids.
La composition peut comprendre un émulsionnant. En particulier, on utilise un émulsionnant hydrosoluble, en particulier ayant une balance HLB (hydrophile-lipophile balance) supérieure ou égale à 10 à 25°C. L'émulsionnant peut être choisi parmi les émulsionnants amphotères, anioniques, cationiques ou non ioniques, et leurs mélanges. Comme émulsionnants amphotères, on peut citer les N-acyl-aminoacides tels que les N-alkyl-aminoacétates et le cocoamphodiacetate disodique et les oxydes d'amines tels que l'oxyde de stéaramine. Comme émulsionnants anioniques, on peut citer les acylgluta-mates tels que le "disodium hydrogenated tallow glutamate" (AMISOFT HS-21^{R} commercialisé par la société Ajinomoto); les acides carboxyliques et leurs sels tels que le stéarate de sodium; les esters phosphoriques et leurs sels tels que le "DEA oleth-10 phosphate"; les sulfoccinates tels que le "Disodium PEG-5 citrate lauryl sulfosuccinate" et le "Disodium ricinoleamido MEA sulfosuccinate"; les alkyl éther sulfates tels que le lauryl éther sulfate de sodium; les sulfosuccinates; les iséthionates. Comme émulsionnants cationiques, on peut citer les alkyl-imidazolidinium tels que l'étho-sulfate d'isos-téaryl-éthylimidonium; les sels d'ammonium tels que le chlorure de N,N,N-triméthyl-1-docosanaminium (Behentrimonium chloride). Comme émulsionnants non ioniques, on peut citer les esters et éthers d'oses tels que le stéarate de sucrose, le cocoate de sucrose, et le mélange de stéarate de sorbitan et de cocoate de sucrose commercialisé par la société ICI sous la dénomination d'Arlatone 2121; les esters d'acides gras (notamment d'acide en C8-C24, et de préférence en C16-C22) et de polyol, notamment de glycérol ou de sorbitol, tels que le stéarate de glycéryle, le stéarate de polyglycéryl-2, le tristéarate de sorbitan, le ricinoléate de glycéryle; les éthers de glycérol; les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné, oxypropyléné de l'alcool laurique à 25 groupes oxyéthylénés et 25 groupes oxypropylénés (nom CTFA "PPG-25 laureth-25") et l'éther oxyéthyléné du mélange d'alcools gras en C12-C15 comportant 7 groupes oxyéthylénés (nom CTFA "C12-15 Pareth-7"); les esters d'acide gras (notamment d'acide en C8-C24, et de préférence en C16-C22) et de polyéthylène glycol (pouvant comprendre de 1 à 150 motifs d'éthylèneglycol) tels que le stéarate de PEG-50 et le stéarate de PEG-40; les copolymères d'oxyde de propylène et d'oxyde d'éthylène tels que ceux vendus sous les dénominations SYNPERONIC par UNIQEMA. Comme émulsionnants siliconés, on peut citer les diméthicone copolyols., telle que celles vendues sous les dénominations "DC2-5695" et « Q2-5220 » par la société Dow Corning, le mélange de cyclométhicone/diméthicone copolyol vendu sous la dénomination "Q2-3225C" par la société Dow Corning, les diméthicone copolyols phosphates tels que celui vendu sous la dénomination PECOSIL PS 100 par la société PHOENIX CHEMICAL. Comme émulsionnant siliconé, on peut également utiliser un diméthicone copolyol benzoate c'est-à-dire un ester partiel d'acide benzoïque et de diméthicone copolyol, ce dernier étant un polymère de diméthylpolysiloxane comportant des chaînes latérales de polyoxyéthylène et/ou de polyoxypropylène. Comme diméthicone copolyol benzoate, on peut utiliser ceux vendus sous la dénomination FINSOLV par la société FINETEX. L'émulsionnant peut être présent dans la composition selon l'invention en une teneur de 0,1% à 30% en poids, par rapport au poids total de la composition, de préférence de 0,5% à 20% en poids, et préférentiellement de 1% à 10% en poids.
De préférence, la composition utilisée selon l'invention comprend une matière colorante additionnelle qui peut être choisie parmi les matières colorantes pulvérulentes comme les pigments, les nacres, les paillettes ou bien encore les matières colorantes hydrosolubles, habituellement utilisés dans les compositions cosmétiques et telles que décrites ci-dessus, et leurs mélanges. La matière colorante additionnelle peut être présente dans la composition selon l'invention en une teneur de 0,1 à 50% en poids, par rapport au poids total de la composition, de préférence de 1 à 40% en poids, et préférentiellement de 10% à 35% en poids.
La composition selon l'invention peut comprendre, en outre, au moins un glycol pour permettre un bon mouillage des pigments, c'est-à-dire faciliter leur mise en oeuvre et leur dispersion homogène (absence d'agglomérat) dans le milieu aqueux lors de la préparation de la composition, puis favoriser la redispersion des pigments lors de la mise en contact du fard à paupières solide avec une phase aqueuse avant application sur les paupières. Le glycol permet un bon mouillage de la peau facilitant l'étalement de la composition sur la paupière. Dans la présente demande, on entend par glycol un diol comprenant de 2 à 8, et de préférence de 2 à 4, atomes de carbone. Le glycol peut être choisi parmi le propylène glycol, l'éthylène glycol, le 1,3-butylène glycol, le dipropylène glycol, et leurs mélanges. Le glycol peut être présent dans la composition en une teneur de 0,1% à 40% en poids, par rapport au poids total de la composition, et de préférence de 5% à 20% en poids.
La composition utilisée selon l'invention peut comprendre des charges qui peuvent être choisies parmi le talc, utilisé sous forme de particules généralement inférieures à 40 microns; les micas, qui sont des aluminosilicates de compositions variées se présentant sous la forme d'écailles ayant des dimensions de 2 à 200 microns, et une épaisseur comprise entre 0,1 à 5 microns, ces micas pouvant être d'origine naturelle telle que la muscovite la margarite, la roscoelithe, la lipidolithe, la biotite ou d'origine synthétique; l'amidon en particulier l'amidon de riz; le kaolin qui peut se présenter sous la forme de particules de forme isotrope ayant des dimensions généralement inférieures à 30 microns; les oxydes de zinc et de titane généralement utilisés sous la forme de particules ayant des dimensions ne dépassant pas quelques microns; le carbonate de calcium, le carbonate ou l'hydrocarbonate de magnésium; la cellulose microcristalline; la silice; les poudres de polymères synthétiques tels que le polyéthylène, les polyesters (l'isophtalate ou le téréphtalate de polyéthylène), les polyamides tels que ceux vendus sous la dénomination commerciale de "Nylon" ou de "Téflon" et les poudres de silicone.
Selon un mode avantageux de réalisation, le fard à paupières utilisé selon l'invention comprend :
- une phase grasse solide, qui comprend au moins une cire; de préférence la phase grasse solide est présente en une teneur de 1% à 30% en poids, notamment de 2% à 20% en poids par rapport au poids total de la composition.
- une phase grasse liquide en une teneur inférieure ou égale à 10% en poids par rapport au poids total de la composition, de préférence inférieure ou égale à 7% en poids, de préférence inférieure ou égale à 5% en poids, mieux inférieure ou égale à 3% en poids et encore mieux inférieure ou égale à 3% en poids; encore mieux la composition de fard à paupière est exempte de phase grasse liquide.
- au moins une charge qui peut être organique ou minérale, de forme sphérique ou lamellaire; qui peut être présentes en une teneur de 0,1 % à 50% en poids par rapport au poids total de la composition, de préférence allant de 1% à 40% en poids.
Préférentiellement, le fard à paupières est anhydre, c'est-à-dire est une composition contenant moins de 2% en poids d'eau (eau ajoutée), voire moins de 0,5% d'eau, notamment moins de 0,2% d'eau, l'eau susceptible d'être présente n'étant pas ajoutée lors de la préparation de la composition mais correspondant à l'eau résiduelle apportée par les ingrédients mélangés.
Le procédé cosmétique de maquillage des matières kératiniques, notamment de la peau du corps ou du visage, des lèvres, des ongles, des cheveux et/ou des cils, comprend l'application sur lesdites matières d'une composition cosmétique telle que définie précédemment.
Ce procédé selon l'invention permet notamment le maquillage desdites matières kératiniques, en particulier des lèvres, du visage, des paupières et/ou des joues, par application d'une composition de rouge à lèvres, de fard à paupières ou à joues, ou de fond de teint, selon l'invention.

L'invention est illustrée plus en détails dans les exemples de réalisation suivants.

### Exemple 1

On mélange à sec, (x) g d'indigo (indigo synthétique; teneur en indigo cristallisé: 95%) avec 25 g de sépiolite (Tolsa S9 grade, (Si)₁₂(Mg)₈(O)₃₀(OH)₄(OH₂)₄, 8H₂O, dimension des tunnels 1,06 x 0,37 nm).

Le cas échéant, le mélange est mélangé/broyé pendant (y) minutes, dans un broyeur de type mortier-pilon, puis le mélange est traité thermiquement dans un four à moufle d'un volume de 8000 cm³, pendant un temps (t) à une température (T). Le chauffage est effectué sous air ambiant, et le mélange est mis dans le four préalablement porté à la température requise.

Le cas échéant, le traitement thermique peut être effectuée en plusieurs étapes, avec une phase de refroidissement à l'air libre, jusqu'à température ambiante (25°C) entre chaque étape.

On laisse ensuite refroidir jusqu'à 25°C la matière colorante ainsi obtenue, qui se présente sous forme de poudre.

On détermine les coordonnées colorimétriques de ladite matière colorante, de la manière suivante: on prépare des poudres compactées sous une pression de 100 bars, dans une coupelle de type FAP247; la quantité de matière colorante est ajustée de manière à remplir complètement la coupelle. La coupelle est re-

## Revendications

1. Procédé cosmétique de maquillage des matières kératiniques, notamment de la peau du corps ou du visage, des lèvres, des ongles, des cheveux et/ou des cils, comprenant l'application sur lesdites matières d'une composition cosmétique comprenant, dans un milieu cosmétiquement acceptable, au moins une matière colorante comprenant une matrice inorganique fibreuse à tunnels et au moins un composé colorant organique incorporé au moins partiellement dans lesdits tunnels,
dans laquelle ledit composé colorant organique est choisi parmi les indigoïdes, et est présent dans la matière colorante en une quantité telle que le rapport pondéral composé colorant organique / matrice fibreuse inorganique est supérieur ou égal à 0,028 (soit 2,8%),
le composé colorant organique et la matrice fibreuse inorganique étant soumis à un traitement thermique effectué :
(i) soit en une seule étape, à une température supérieure ou égale à 320°C, pendant un temps supérieur ou égal à 15 minutes,
(ii) soit en une seule étape, à une température supérieure ou égale à 250°C, pendant un temps supérieur ou égal à 45 minutes ;
(iii) soit en deux étapes, la première étape étant effectuée à une température supérieure ou égale à 250°C, pendant un temps supérieur ou égal à 10 minutes, la seconde étape étant effectuée à une température supérieure ou égale à 250°C, pendant un temps supérieur ou égal à 5 minutes ;
(iv) soit en trois étapes, chaque étape étant effectuée à une température supérieure ou égale à 320°C, pendant un temps supérieur ou égal à 4 minutes.

2. Procédé selon la revendication 1, dans lequel la matrice inorganique fibreuse à tunnels est une argile fibreuse, notamment de type sépiolite ou palygorskite.

3. Procédé selon l'une des revendications précédentes, dans lequel la matrice inorganiques fibreuse à tunnels est une sépiolite.

4. Procédé selon l'une des revendications précédentes, dans lequel le composé colorant organique est de l'indigo, seul ou en mélange avec au moins un colorant organique additionnel de la famille des indigoïdes, choisi parmi l'indirubine, l'indigotine, les halogéno-indigo tels que le dichloroindigo, le dibromoindigo, les thioindigos, les acétates d'indigo.

5. Procédé selon l'une des revendications précédentes, dans lequel les composés colorants organiques sont tels qu'au moins deux de leurs dimensions sont inférieures ou égales à, respectivement, 1,1 nm et 0,57 nm, la troisième dimension pouvant être quelconque.

6. Procédé selon l'une des revendications précédentes, dans lequel le composé colorant organique est présent dans la matière colorante en une quantité telle que le rapport pondéral initial composé colorant organique / matrice fibreuse inorganique est compris entre 0,0285 (2,85%) et 0,20 (20%), encore mieux entre 0,029 (2,9%) et 0,15 (15%), voire entre 0,03 (3%) et 0,13 (13%), préférentiellement entre 0,032 (3,2%) et 0,08 (8%).

7. Procédé selon l'une des revendications précédentes, dans lequel les composés colorants organiques et la matrice fibreuse inorganique sont soumis à une étape de mélange/malaxage, qui peut être effectuée pendant 10 secondes à 72 heures, notamment 1 à 120 minutes, encore mieux 10 à 100 minutes.

8. Procédé selon la revendication 1, dans lequel le traitement thermique est effectué en une seule étape, à une température supérieure ou égale à 320°C, notamment comprise entre 320°C et 600°C, de préférence comprise entre 340°C et 550°C, pendant un temps supérieur ou égal à 15 minutes, notamment compris entre 15 et 45 minutes, de préférence entre 18 et 40 minutes.

9. Procédé selon la revendication 1, dans lequel le traitement thermique est effectué en une seule étape, à une température supérieure ou égale à 250°C, notamment comprise entre 250°C et 320°C, de préférence comprise entre 260°C et 300°C, pendant un temps supérieur ou égal à 45 minutes, notamment compris entre 45 et 120 minutes, de préférence entre 50 et 90 minutes.

10. Procédé selon la revendication 1, dans lequel le traitement thermique est effectué en deux étapes,
la première étape étant effectuée à une température supérieure ou égale à 250°C, notamment comprise entre 250°C et 320°C, de préférence comprise entre 260°C et 300°C, pendant un temps supérieur ou égal à 10 minutes, notamment compris entre 10 et 120 minutes, de préférence entre 15 et 90 minutes;
la seconde étape étant effectuée à une température supérieure ou égale à 250°C, notamment comprise entre 250°C et 350°C, de préférence comprise entre 270°C et 320°C, pendant un temps supérieur ou égal à 5 minutes, notamment compris entre 5 et 90 minutes, de préférence entre 10 et 60 minutes.

11. Procédé selon la revendication 1, dans lequel le traitement thermique est effectué en trois étapes, chaque étape étant effectuée à une température supérieure ou égale à 320°C, notamment comprise entre 320°C et 600°C, de préférence comprise entre 340°C et 550°C, pendant un temps supérieur ou égal à 4 minutes, notamment compris entre 4 et 40 minutes, de préférence entre 5 et 30 minutes.

12. Procédé selon l'une des revendications précédentes, dans laquelle les matières colorantes sont présentes dans la composition cosmétique à raison de 0,1 à 70% en poids, notamment 0,5 à 50% en poids, voire 1 à 40% en poids, préférentiellement 5 à 35% en poids, par rapport au poids total de la composition cosmétique.

13. Procédé selon l'une des revendications précédentes, dans lequel la composition se présente sous la forme d'un produit de maquillage de la peau, du corps ou du visage, des lèvres, des cils, des sourcils, des cheveux, du cuir cheveu ou des ongles.

14. Procédé selon l'une des revendications précédentes, dans lequel la composition se présente sous forme de composition de maquillage, notamment de mascara, de rouge à lèvres, de fard à joues, de fard à paupières, de fond de teint.

## Patentansprüche

1. Kosmetisches Verfahren zum Make-up von Keratinmaterialien, insbesondere der Körper- oder Gesichtshaut, der Lippen, der Nägel, der Haare und/oder der Wimpern, bei dem man auf die Materialien eine kosmetische Zusammensetzung aufbringt, die in einem kosmetisch unbedenklichen Medium mindestens ein Farbmittel mit einer faserförmigen anorganischen Matrix mit Tunneln und mindestens eine zumindest teilweise in die Tunnel inkorporierte organische Farbstoffverbindung umfasst,
wobei die organische Farbstoffverbindung aus Indigoiden ausgewählt ist und in dem Farbmittel in einer solchen Menge vorliegt, dass das Gewichtsverhältnis von organischer Farbstoffverbindung zu faserförmiger anorganischer Matrix größer oder gleich 0,028 (d. h. 2,8 %) ist,
wobei die organische Farbstoffverbindung und die faserförmige anorganische Matrix einer Wärmebehandlung unterworfen werden, die
(i) entweder in einem einzigen Schritt bei einer Temperatur größer öder gleich 320 °C über einen Zeitraum größer oder gleich 15 Minuten
(ii) oder in einem einzigen Schritt bei einer Temperatur größer oder gleich 250 °C über einen Zeitraum größer oder gleich 45 Minuten;
(iii) oder in zwei Schritten, wobei der erste Schritt bei der Temperatur größer oder gleich 250 °C über einen Zeitraum größer oder gleich 10 Minuten durchgeführt wird und der zweite Schritt bei der Temperatur größer oder gleich 250 °C über einen Zeitraum größer oder gleich 5 Minuten durchgeführt wird;
(iv) oder in drei Schritten, wobei jeder Schritt bei einer Temperatur größer oder gleich 320 °C über einen Zeitraum größer oder gleich 4 Minuten durchgeführt wird;
durchgeführt wird.

2. Verfahren nach Anspruch 1, wobei es sich bei der faserförmigen anorganischen Matrix mit Tunneln um einen faserförmigen Ton, insbesondere vom Sepiolith- oder Palygorskit-Typ, handelt.

3. Verfahren nach einem der vorhergehenden Ansprüche, wobei es sich bei der faserförmigen anorganischen Matrix mit Tunneln um einen Sepiolith handelt.

4. Verfahren nach einem der vorhergehenden Ansprüche, wobei es sich bei der organischen Farbstoffverbindung um Indigo, allein oder als Mischung mit mindestens einem zusätzlichen organischen Farbstoff aus der Familie der Indigoide, der aus Indirubin, Indigotin, Halogenindigos wie Dichlorindigo, Dibromindigo, Thioindigos oder Indigoacetaten ausgewählt ist, handelt.

5. Verfahren nach einem der vorhergehenden Ansprüche, wobei die organischen Farbstoffverbindungen so beschaffen sind, dass mindestens zwei ihrer Abmessungen kleiner oder gleich 1,1 nm bzw. 0,57 nm sind, wobei die dritte Abmessung einen beliebigen Wert haben kann.

6. Verfahren nach einem der vorhergehenden Ansprüche, wobei die organische Farbstoffverbindung in dem Farbmittel in einer solchen Menge vorliegt, dass das Anfangsgewichtsverhältnis von organischer Farbstoffverbindung zu faserförmiger anorganischer Matrix zwischen 0,0285 (2,85 %) und 0,20 (20 %), noch besser zwischen 0,029 (2,9 %) und 0,15 (15 %) oder sogar zwischen 0,03 (3 %) und 0,13 (13 %) und vorzugsweise zwischen 0,032 (3,2 %) und 0,08 (8 %) liegt.

7. Verfahren nach einem der vorhergehenden Ansprüche, wobei die organischen Farbstoffverbindungen und die faserförmige anorganische Matrix einer Misch-/Knet-Stufe unterworfen werden, die über einen Zeitraum von 10 Sekunden bis 72 Stunden, insbesondere 1 bis 120 Minuten und noch besser 10 bis 100 Minuten durchgeführt werden kann.

8. Verfahren nach Anspruch 1, bei dem die Wärmebehandlung in einem einzigen Schritt bei einer Temperatur größer oder gleich 320 °C, insbesondere zwischen 320 °C und 600 °C, vorzugsweise zwischen 340 °C und 550 °C, über einen Zeitraum größer oder gleich 15 Minuten, insbesondere zwischen 15 und 45 Minuten, vorzugsweise zwischen 18 und 40 Minuten, durchgeführt wird.

9. Verfahren nach Anspruch 1, bei dem die Wärmebehandlung in einem einzigen Schritt bei einer Temperatur größer oder gleich 250 °C, insbesondere zwischen 250 °C und 320 °C, vorzugsweise zwischen 260 °C und 300 °C, über einen Zeitraum größer oder gleich 45 Minuten, insbesondere zwischen 45 und 120 Minuten, vorzugsweise zwischen 50 und 90 Minuten, durchgeführt wird.

10. Verfahren nach Anspruch 1, bei dem die Wärmebehandlung in zwei Schritten durchgeführt wird,
wobei der erste Schritt bei einer Temperatur größer oder gleich 250 °C, insbesondere zwischen 250 °C und 320 °C, vorzugsweise zwischen 260 °C und 300 °C, über einen Zeitraum größer oder gleich 10 Minuten, insbesondere zwischen 10 und 120 Minuten, vorzugsweise zwischen 15 und 90 Minuten durchgeführt wird;
wobei der zweite Schritt bei einer Temperatur größer oder gleich 250 °C, insbesondere zwischen 250 °C und 350 °C, vorzugsweise zwischen 270 °C und 320 °C, über einen Zeitraum größer oder gleich 5 Minuten, insbesondere zwischen 5 und 90 Minuten, vorzugsweise zwischen 10 und 60 Minuten, durchgeführt wird.

11. Verfahren nach Anspruch 1, bei dem die Wärmebehandlung in drei Schritten durchgeführt wird, wobei jeder Schritt bei einer Temperatur größer oder gleich 320 °C, insbesondere zwischen 320 °C und 600 °C, vorzugsweise zwischen 340 °C und 550 °C, über einen Zeitraum größer oder gleich 4 Minuten, insbesondere zwischen 4 und 40 Minuten, vorzugsweise zwischen 5 und 30 Minuten, durchgeführt wird.

12. Verfahren nach einem der vorhergehenden Ansprüche, bei dem die Farbmittel in der kosmetischen Zusammensetzung in einem Anteil von 0,1 bis 70 Gew.-%, insbesondere 0,5 bis 50 Gew.-% oder sogar 1 bis 40 Gew.-% und vorzugsweise 5 bis 35 Gew.-%, bezogen auf das Gesamtgewicht der kosmetischen Zusammensetzung, vorliegen.

13. Verfahren nach einem der vorhergehenden Ansprüche, wobei die Zusammensetzung in Form eines Make-up-Produkts für die Körper- oder Gesichtshaut, die Lippen, die Wimpern, die Augenbrauen, das Haars, die Kopfhaut oder die Nägel vorliegt.

14. Verfahren nach einem der vorhergehenden Ansprüche, wobei die Zusammensetzung in Form einer Make-up-Zusammensetzung, insbesondere eines Mascaras, eines Lippenstifts, eines Rouges, eines Lidschattens oder einer Make-up-Grundlage, vorliegt.

## Claims

1. Cosmetic method for making up keratin materials, notably the skin of the body or of the face, lips, nails, hair and/or of the eyelashes, comprising the application on said materials of a cosmetic composition comprising, in a cosmetically acceptable medium, at least one colourant comprising an inorganic fibrous matrix with tunnels and at least one organic dye compound incorporated at least partially in said tunnels,
in which said organic dye compound is selected from the indigoids, and is present in the colourant in an amount such that the weight ratio of organic dye compound to inorganic fibrous matrix is greater than or equal to 0.028 (i.e. 2.8%),
the organic dye compound and the inorganic fibrous matrix being subjected to a heat treatment carried out:
(i) either in a single stage, at a temperature greater than or equal to 320°C, for a time greater than or equal to 15 minutes;
(ii) or in a single stage, at a temperature greater than or equal to 250°C, for a time greater than or equal to 45 minutes;
(iii) or in two stages, the first stage being carried out at a temperature greater than or equal to 250°C, for a time greater than or equal to 10 minutes,
the second stage being carried out at a temperature greater than or equal to 250°C, for a time greater than or equal to 5 minutes;
(iv) or in three stages, each stage being carried out at a temperature greater than or equal to 320°C, for a time greater than or equal to 4 minutes.

2. Process according to Claim 1, in which the inorganic fibrous matrix with tunnels is a fibrous clay, notably of the sepiolite or palygorskite type.

3. Process according to either of the preceding claims, in which the inorganic fibrous matrix with tunnels is a sepiolite.

4. Process according to one of the preceding claims, in which the organic dye compound is indigo, alone or mixed with at least one additional organic dye from the indigoid family, selected from indirubin, indigotin, haloindigos such as dichloroindigo, dibromoindigo, thioindigos, or indigo acetates.

5. Process according to one of the preceding claims, in which the organic dye compounds are such that at least two of their dimensions are less than or equal to, respectively, 1.1 nm and 0.57 nm, with the third dimension being able to have any value.

6. Process according to one of the preceding claims, in which the organic dye compound is present in the colourant in an amount such that the initial weight ratio of organic dye compound to inorganic fibrous matrix is between 0.0285 (2.85%) and 0.20 (20%), better still between 0.029 (2.9%) and 0.15 (15%), or even between 0.03 (3%) and 0.13 (13%), preferably between 0.032 (3.2%) and 0.08 (8%).

7. Process according to one of the preceding claims, in which the organic dye compounds and the inorganic fibrous matrix are subjected to a stage of mixing/kneading, which can be carried out for 10 seconds to 72 hours, notably 1 to 120 minutes, better still 10 to 100 minutes.

8. Process according to Claim 1, in which the heat treatment is carried out in a single stage, at a temperature greater than or equal to 320°C, notably between 320°C and 600°C, preferably between 340°C and 550°C, for a time greater than or equal to 15 minutes, notably between 15 and 45 minutes, preferably between 18 and 40 minutes.

9. Process according to Claim 1, in which the heat treatment is carried out in a single stage, at a temperature greater than or equal to 250°C, notably between 250°C and 320°C, preferably between 260°C and 300°C, for a time greater than or equal to 45 minutes, notably between 45 and 120 minutes, preferably between 50 and 90 minutes.

10. Process according to Claim 1, in which the heat treatment is carried out in two stages,
the first stage being carried out at a temperature greater than or equal to 250°C, notably between 250°C and 320°C, preferably between 260°C and 300°C, for a time greater than or equal to 10 minutes, notably between 10 and 120 minutes, preferably between 15 and 90 minutes;
the second stage being carried out at a temperature greater than or equal to 250°C, notably between 250°C and 350°C, preferably between 270°C and 320°C, for a time greater than or equal to 5 minutes, notably between 5 and 90 minutes, preferably between 10 and 60 minutes.

11. Process according to Claim 1, in which the heat treatment is carried out in three stages, each stage being carried out at a temperature greater than or equal to 320°C, notably between 320°C and 600°C, preferably between 340°C and 550°C, for a time greater than or equal to 4 minutes, notably between 4 and 40 minutes, preferably between 5 and 30 minutes.

12. Process according to one of the preceding claims, in which the colourants are present in the cosmetic composition in a proportion of 0.1% to 70% by weight, notably 0.5% to 50% by weight, or even 1% to 40% by weight, preferably 5% to 35% by weight, relative to the total weight of the cosmetic composition.

13. Process according to one of the preceding claims, in which the composition is in the form of a product for making up the skin, of the body or of the face, lips, eyelashes, eyebrows, hair, scalp or nails.

14. Process according to one of the preceding claims, in which the composition is in the form of a makeup composition, notably of mascara, of lipstick, of face powder, of eyeshadow, or of foundation.
